# EUROPEAN PATENT SPECIFICATION

(11) **EP 3 960 090 B1**
(45) Date of publication and mention of the grant of the patent: **08.01.2025**
(21) Application number: 19926005.0
(22) Date of filing: 09.12.2019
(51) Int. Cl.: A61B 8/00

(54) **TWO-PROBE WIRELESS ULTRASONIC DEVICE, CONTROL METHOD THEREFOR, AND CONTROL SYSTEM THEREOF**
DRAHTLOSE ULTRASCHALLVORRICHTUNG MIT ZWEI SONDEN, STEUERUNGSVERFAHREN DAFÜR UND STEUERUNGSSYSTEM DAFÜR
DISPOSITIF ULTRASONORE SANS FIL À DEUX SONDES, SON PROCÉDÉ DE COMMANDE ET SON SYSTÈME DE COMMANDE

(30) Priority: 24.04.2019 CN 201910335638
(43) Date of publication of application: 02.03.2022
(73) Proprietor: Vinno Technology (Suzhou) Co., Ltd., Jiangsu 215123 (CN)
(72) Inventor: GUO, Mingkun, Suzhou Industrial Park Jiangsu 215123 (CN)
(74) Representative: Winter, Brandl - Partnerschaft mbB
(86) International application number: PCT/CN2019/123868
(87) International publication number: WO 2020/215740

(56) References cited:
- CN-A- 101 366 662
- CN-A- 101 742 968
- CN-A- 106 999 149
- CN-A- 109 893 170
- CN-U- 201 629 739
- US-A1- 2013 169 507
- No further relevant documents disclosed

## Description

### TECHNICAL FIELD

The present application relates to the field of medical ultrasonic diagnostic imaging, and particularly to a dual-probe wireless ultrasonic device, a control method and a control system thereof.

### BACKGROUND

The dual-probe ultrasonic device is a medical device manufactured based on the principle of ultrasound, which is used in conjunction with an ultrasonic machine and is configured to transmit and receive ultrasonic waves during the ultrasonic detection process. A dual-probe wireless ultrasonic device usually includes two probes. The types of the two probes can be the same or different. The type of the probe includes, for example, a linear array probe, an arc array probe, and a phased array probe.

The dual-probe ultrasonic device includes: two sets of ultrasonic probes provided on a probe body and having different installation positions, a control board respectively connected to the two sets of ultrasonic probes and provided inside the probe body, and a communication device for transmitting images.

In the prior art, a USB cable is usually connected to the outside of the probe body to connect with an external communication device for image transmission. With the increase in user demands, the demand for dual-probe wireless ultrasonic devices has become more widespread. In this device, the communication device is an antenna which can transmit wireless signals to communicate with external devices and then transmit images.

For the dual-probe wireless ultrasonic device, there are mainly two manners to install the antenna in the prior art. One is to install the antenna on an ultrasonic probe side. This installation manner is subject to requirements of synchronization signal transmission of the ultrasonic probe. The installation of the antenna on the ultrasonic probe side may affect the image quality. The other manner is to install the antenna on a main body housing. For this solution of installing the antenna on the main body housing, if the user holds the probe body during use, the antenna signal transmission is also affected.

The document CN 101366662 A discloses a multi-headed imaging probe and an imaging system using the same. Specifically provided is the diagnostic imaging system that includes an image acquisition component, a transmitter operatively coupled to the image acquisition component to transmit a signal therefrom, and a beamformer operatively coupled to the image acquisition component to receive image data therefrom. Also included is a processor configured to assemble images from the acquired image data and a display configured to display the images. The image acquisition component includes a multi-headed probe that has a plurality of transducers configured to permit a change of active transducers during an imaging session without a change of the image acquisition component.

The document CN 101742968 A discloses a wireless ultrasound probe having a probe case enclosing a transducer array, a probe controller, and a transceiver which wirelessly receives control signals from and transmits image signals to a host system. Mounted on the probe case is a liquid-tight user interface including basic probe user controls such as directional controls, an image freeze control, and an image save control. The user interface may be fabricated as a touch panel LCD or OLED display. The probe may alternatively be controlled by a separate user interface which wirelessly transmits control signals to the host system or the probe. Either user interface may also include a display such as battery charge and signal strength indicators.

### SUMMARY

The present application provides a dual-probe wireless ultrasonic device, a control method and a control system thereof.

In order to achieve the above application purposes, a dual-probe wireless ultrasonic device is provided in an embodiment of the present application, including: two sets of ultrasonic probes arranged on a probe body and having different installation positions, and a control board communicatively connected with the two sets of ultrasonic probes respectively and arranged inside the probe body;

the dual-probe wireless ultrasonic device further includes: two sets of antennas for data transmission and communicatively connected with the control board;

the two sets of ultrasonic probes comprise: a first ultrasonic probe and a second ultrasonic probe;

the two sets of antennas comprising: a first antenna arranged on the second ultrasonic probe and communicatively connected with the first ultrasonic probe, and a second antenna arranged on the first ultrasonic probe and communicatively connected with the second ultrasonic probe.

As an improvement of an embodiment of the present application, a material of the probe body is a metal material and/or a plastic material.

As an improvement of an embodiment of the present application, the two sets of ultrasonic probes are respectively arranged at two opposite end portions of the probe body.

As an improvement of an embodiment of the present application, at least one of the two sets of antennas is arranged away from the probe body.

As an improvement of an embodiment of the present application, each ultrasonic probe includes: a housing configured to receive a functional component of the ultrasonic probe, and at least one of the two sets of antennas is arranged adjacent to an inner wall surface of the housing.

As an improvement of an embodiment of the present application, at least one of the two sets of antennas is arranged on the inner wall surface of the housing.

As an improvement of an embodiment of the present application, at least one of the two sets of antennas is glued and fixed on the inner wall surface of the housing, or fixed on the inner wall surface of the housing in a snap-fit manner.

As an improvement of an embodiment of the present application, the dual-probe wireless ultrasonic device further includes: a battery arranged inside the probe body.

In order to achieve the above invention purposes, a method for controlling a dual-probe wireless ultrasonic device of the above embodiment is provided in an embodiment of the present application, including:
monitoring a currently activated ultrasonic probe of the first and second ultrasonic probes in real time after turning on the dual-probe wireless ultrasonic device;
if the first ultrasonic probe is monitored and activated, turning on a first antenna and turning off a second antenna;
if the second ultrasonic probe is monitored and activated, turning on the second antenna and turning off the first antenna.

In order to achieve the above invention purposes, a system for controlling a dual-probe wireless ultrasonic device of the above embodiment is provided in an embodiment of the present application, including:
an operation status monitoring module, configured to monitor a currently activated ultrasonic probe of the first and second ultrasonic probes in real time after the dual-probe wireless ultrasonic device is turned on;
a control conversion module, configured to control turn-on of an antenna of the first and second antennas through the activated ultrasonic probe;
if the first ultrasonic probe is monitored and activated, a first antenna is turned on and a second antenna is turned off;
if the second ultrasonic probe is monitored and activated, the second antenna is turned on and the first antenna is turned off.

Compared to the prior art, the present application has the beneficial effects that: the dual-probe wireless ultrasonic device of the present application can wirelessly support the operation of dual-acoustic probes, and when any probe operates, the transmission of the probe signal is not interfered, thereby achieving an image with a better quality.

### BRIEF DESCRIPTION OF THE DRAWINGS

FIG. 1 is a schematic diagram illustrating an overall structure of a dual-probe wireless ultrasonic device.
FIG. 2 is a schematic diagram illustrating an exploded structure of FIG. 1.
FIG. 3 is a cross-sectional view of FIG. 1.
FIG. 4 is a schematic flowchart showing a method for controlling a dual-probe wireless ultrasonic device according to an embodiment of the present application.
FIG. 5 is a schematic diagram illustrating modules of a system for controlling a dual-probe wireless ultrasonic device according to an embodiment of the present application.

### DETAILED DESCRIPTION

The present application will be described in detail with reference to specific embodiments shown in accompanying drawings. However, these embodiments do not limit the present application; and transformations in the structure, method, or function made by those skilled in the art based on these embodiments are all included in the protection scope of the present application.

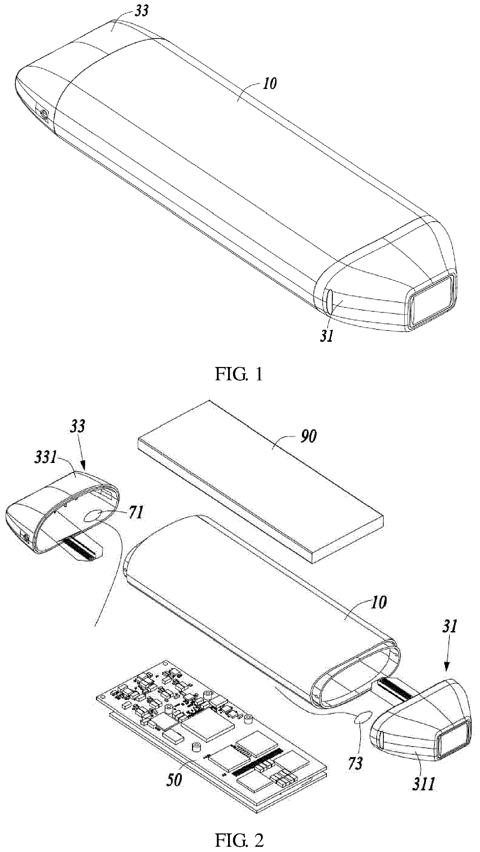

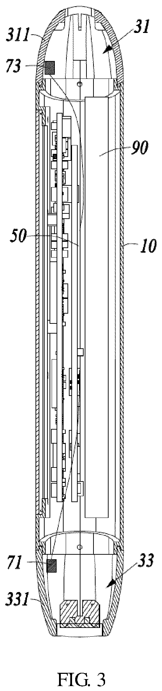

Referring to FIGS. 1-3, a dual-probe wireless ultrasonic device is provided in an embodiment of the present application, which includes: two sets of ultrasonic probes provided on a probe body 10 and having different installation positions, a control board 50 respectively communicatively connected with the two sets of ultrasonic probes and provided inside the probe body 10, and two sets of antennas for a data transmission and communicatively connected with the control board 50. The ultrasonic probes include a first ultrasonic probe 31 and a second ultrasonic probe 33 provided at different positions on the probe body 10. The antennas include: a first antenna 71 provided on the second ultrasonic probe 33 and communicatively connected with the first ultrasonic probe 31, and a second antenna 73 provided on the first ultrasonic probe 31 and communicatively connected with the second ultrasonic probe 33.

It should be noted that the types of the first ultrasonic probe 31 and the second ultrasonic probe 33 can be the same or different, which can be both one of a linear array probe, an arc array probe, and a phased array probe. In a specific implementation of the present application, the first ultrasonic probe 31 is an arc array probe, and the second ultrasonic probe 33 is a phased array probe.

In an embodiment of the present application, since the antenna is provided by avoiding the probe body 10, a material of the probe body 10 does not need to be limited, which can be, for example, ABS, PC, plastic, metal, etc. In a preferred embodiment of the present application, the material of the probe body 10 is a metal material and/or a plastic material. Therefore, when the dual-probe wireless ultrasonic device has a requirement to ground, and the material of the probe body 10 is set to the metal or electroplating within a plastic housing, etc., without screening the transmission of the antenna signal.

Correspondingly, in a preferred embodiment of the present application, the material of the probe body 10 is the metal material. In such a manner, while meeting the requirement of grounding the dual-probe wireless ultrasonic device, the product can be made lighter and thinner, and has a certain texture. Further, the transmission of the antenna signal is not affected either.

In embodiments of the present application, the positions of the two probes are not specifically limited. For example, the two ultrasonic probes are arranged at an acute angle, a right angle, or an obtuse angle between them. In a preferred embodiment of the present application, the two sets of ultrasonic probes are respectively arranged at two opposite end portions of the probe body 10.

In embodiments of the present application, the installation position of any set of antennas corresponding to the ultrasonic probe can be freely selected, for example, can be arranged on the outside and/or inside of the ultrasonic probe.

It should be appreciated that, for each ultrasonic probe, the farther away the antenna communicatively connected with the ultrasonic probe, the better the communication effect. In a preferred embodiment of the present application, the two sets of antennas are arranged away from the probe body 10, to increase a distance between the corresponding ultrasonic probe and the antenna as far as possible to ensure the communication effect.

In a preferred implementation of the present application, the first ultrasonic probe 31 includes a housing 311 configured to receive a functional component of the first ultrasonic probe; the second ultrasonic probe 33 includes a housing 331 configured to receive a functional component of the second ultrasonic probe; the materials of the housings (311, 331) are all plastic materials. Preferably, the two sets of antennas can be selectively arranged inside the corresponding ultrasonic probes respectively. The functional component is, for example, a transducer, etc., which is not described in detail here.

In an embodiment of the present application, the two sets of antennas (71, 73) can be selectively arranged adjacent to inner wall surfaces of the corresponding housings (331, 311) respectively.

In a further embodiment of the present application, the two sets of antennas (71, 73) can be selectively arranged on the inner wall surfaces of the corresponding housings (331, 311) respectively.

In a specific embodiment of the present application, the two sets of antennas (71, 73) can be fixed on the inner wall surfaces of the corresponding housings (331, 311) respectively in a variety of ways, for example, can be glued and fixed on the inner wall surfaces of the housings respectively, or fixed on the inner wall surfaces of the housings respectively in a snap-fit manner, which will not be described in detail here.

In a specific embodiment of the present application, the first antenna 71 is glued and fixed on the inner wall surface of the housing 331 of the second ultrasonic probe 33, and is arranged adjacent to the housing 331 and away from the end portion of the probe body 10. The second antenna is glued and fixed on the inner wall surface of the housing 311 of the first ultrasonic probe 31, and is arranged adjacent to the housing 311 and away from the end portion of the probe body 10.

In addition, in a preferred embodiment of the present application, the first antenna 71 and the second antenna 73 are both communicatively connected with the control board 50 through a cable, and the control board 50 controls the conductions of the two sets of antennas during the operation of the dual-probe wireless ultrasonic device. The specific conduction process will be described in detail below.

In a preferred embodiment of the present application, the dual-probe wireless ultrasonic device further includes: a battery 90 arranged inside the probe body. The battery 90 is configured to provide power to the entire device to satisfy various application scenarios, which will not be detailed here.

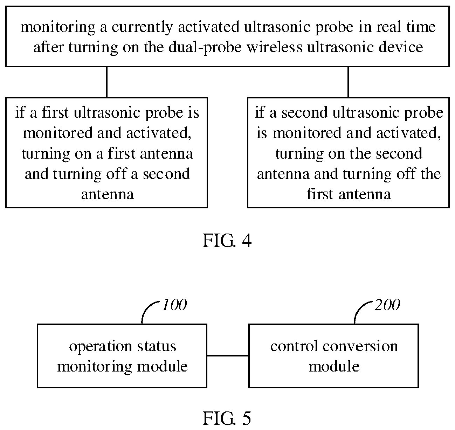

As shown in FIG. 4, in an embodiment of the present application, a method for controlling a dual-probe wireless ultrasonic device as described above is provided. The method includes: if the first ultrasonic probe is monitored and activated, the first antenna is turned on while the second antenna is turned off; if the second ultrasonic probe is monitored and activated, the second antenna is turned on while the first antenna is turned off; the first antenna is arranged on the second ultrasonic probe, and the second antenna is arranged on the first ultrasonic probe. In this way, when the first ultrasonic probe operates, the second antenna arranged thereon is automatically turned off, and the first antenna which is away from the first ultrasonic probe and is arranged on the second ultrasonic probe is activated; when the second ultrasonic probe operates, the first antenna arranged thereon is automatically turned off, and the second antenna which is away from the second ultrasonic probe and is arranged on the first ultrasonic probe is activated; thus, it can be ensured that each ultrasonic probe is not interfered by a signal from the antenna arranged thereon when operating; at the same time, because the antenna is arranged on the ultrasonic probe, the user can also hold any position on the probe body when using the wireless ultrasonic device, which is convenient for the user to operate.

As shown in FIG. 5, the present application also provides a system for controlling a dual-probe wireless ultrasonic device as described above, the system includes: an operation state monitoring module 100 and a control conversion module 200. The working status monitoring module 100 is configured to monitor a currently activated ultrasonic probe in real time after the dual-probe wireless ultrasonic device is turned on. The control conversion module 200 is configured to control turn-on of the antenna through the activated ultrasonic probe; if the first ultrasonic probe is monitored and activated, the first antenna is turned on while the second antenna is turned off; if the second ultrasonic probe is monitored and activated, the second antenna is turned on while the first antenna is turned off. The first antenna is arranged on the second ultrasonic probe, and the second antenna is arranged on the first ultrasonic probe.

In conclusion, the dual-probe wireless ultrasonic device in the present application can wirelessly support dual-acoustic probe operation; and when any probe operates, the transmission of the probe signal is not interfered, thereby obtaining images with a higher quality.

It should be understood that although this specification is described in accordance with the embodiments, not each embodiment only includes one independent technical solution. This narration mode in the specification is merely for clarity, and those skilled in the art should regard the specification as a whole. The technical solutions in the embodiments can also be appropriately combined to form other embodiments that can be understood by those skilled in the art.

The series of detailed descriptions listed above are merely specific descriptions of feasible embodiments of the present application, which are not intended to limit the scope of protection of the present application.

## Claims

1. A dual-probe wireless ultrasonic device, comprising: two sets of ultrasonic probes arranged on a probe body (10) and having different installation positions, and a control board (50) communicatively connected with the two sets of ultrasonic probes respectively and arranged inside the probe body (10);
wherein the dual-probe wireless ultrasonic device further comprises: two sets of antennas for data transmission and communicatively connected with the control board;
wherein, the two sets of ultrasonic probes comprise: a first ultrasonic probe (31) and a second ultrasonic probe (33);
the two sets of antennas comprising: a first antenna (71) arranged on the second ultrasonic probe (33) and communicatively connected with the first ultrasonic probe (31), and a second antenna (73) arranged on the first ultrasonic probe (31) and communicatively connected with the second ultrasonic probe (33).

2. The dual-probe wireless ultrasonic device according to claim 1, wherein a material of the probe body (10) is a metal material and/or a plastic material.

3. The dual-probe wireless ultrasonic device according to claim 1, wherein the two sets of ultrasonic probes are respectively arranged at two opposite end portions of the probe body (10).

4. The dual-probe wireless ultrasonic device according to claim 1, wherein at least one of the two sets of antennas is arranged away from the probe body (10).

5. The dual-probe wireless ultrasonic device according to claim 1, wherein each ultrasonic probe comprises: a housing (311, 331) configured to receive a functional component of the ultrasonic probe, and at least one of the two sets of antennas is arranged adjacent to an inner wall surface of the housing (311, 331).

6. The dual-probe wireless ultrasonic device according to claim 5, wherein at least one of the two sets of antennas is arranged on the inner wall surface of the housing (311, 331).

7. The dual-probe wireless ultrasonic device according to claim 6, wherein at least one of the two sets of antennas is glued and fixed on the inner wall surface of the housing (311, 331), or fixed on the inner wall surface of the housing (311, 331) in a snap-fit manner.

8. The dual-probe wireless ultrasonic device according to claim 6, further comprising: a battery (90) arranged inside the probe body (10).

9. A method for controlling a dual-probe wireless ultrasonic device according to claim 1 comprising:
monitoring a currently activated ultrasonic probe of the first and second ultrasonic probes in real time after turning on the dual-probe wireless ultrasonic device;
if the first ultrasonic probe is monitored and activated, turning on the first antenna and turning off the second antenna;
if the second ultrasonic probe is monitored and activated, turning on the second antenna and turning off the first antenna.

10. A system for controlling a dual-probe wireless ultrasonic device according to claim 1 comprising:
an operation status monitoring module (100), configured to monitor a currently activated ultrasonic probe of the first and second ultrasonic probes in real time after the dual-probe wireless ultrasonic device is turned on;
a control conversion module (200), configured to control turn-on of an antenna of the first and second antennas through the activated ultrasonic probe;
wherein, if the first ultrasonic probe is monitored and activated, the first antenna is turned on and the second antenna is turned off;
if the second ultrasonic probe is monitored and activated, the second antenna is turned on and the first antenna is turned off.

## Patentansprüche

1. Drahtlose Doppelsonden-Ultraschallvorrichtung mit: zwei Sätzen von Ultraschallsonden, die an einem Sondenkörper (10) angeordnet sind und unterschiedliche Installationspositionen haben, und einer Steuerplatine (50), die kommunikativ mit den beiden Sätzen von Ultraschallsonden verbunden ist und im Inneren des Sondenkörpers (10) angeordnet ist,
wobei die drahtlose Doppelsonden-Ultraschallvorrichtung ferner Folgendes aufweist: zwei Sätze von Antennen für die Datenübertragung, die kommunikativ mit der Steuerplatine verbunden sind;
wobei die zwei Sätze von Ultraschallsonden Folgendes aufweisen: eine erste Ultraschallsonde (31) und eine zweite Ultraschallsonde (33);
die zwei Sätze von Antennen Folgendes aufweisen: eine erste Antenne (71), die an der zweiten Ultraschallsonde (33) angeordnet und mit der ersten Ultraschallsonde (31) kommunikativ verbunden ist, und eine zweite Antenne (73), die an der ersten Ultraschallsonde (31) angeordnet und mit der zweiten Ultraschallsonde (33) kommunikativ verbunden ist.

2. Drahtlose Doppelsonden-Ultraschallvorrichtung nach Anspruch 1, wobei ein Material des Sondenkörpers (10) ein Metallmaterial und/oder ein Kunststoffmaterial ist.

3. Drahtlose Doppelsonden-Ultraschallvorrichtung nach Anspruch 1, wobei die zwei Sätze von Ultraschallsonden jeweils an zwei gegenüberliegenden Endabschnitten des Sondenkörpers (10) angeordnet sind.

4. Drahtlose Doppelsonden-Ultraschallvorrichtung nach Anspruch 1, wobei mindestens einer der zwei Sätze von Antennen entfernt vom Sondenkörper (10) angeordnet ist.

5. Drahtlose Doppelsonden-Ultraschallvorrichtung nach Anspruch 1, wobei jede Ultraschallsonde Folgendes aufweist: ein Gehäuse (311, 331), das so konfiguriert ist, dass es eine Funktionskomponente der Ultraschallsonde aufnimmt, und mindestens einer der zwei Sätze von Antennen angrenzend an eine Innenwandfläche des Gehäuses (311, 331) angeordnet ist.

6. Drahtlose Doppelsonden-Ultraschallvorrichtung nach Anspruch 5, wobei mindestens einer der zwei Sätze von Antennen an der Innenwandfläche des Gehäuses (311, 331) angeordnet ist.

7. Drahtlose Doppelsonden-Ultraschallvorrichtung nach Anspruch 6, wobei mindestens einer der zwei Sätze von Antennen auf die Innenwandfläche des Gehäuses (311, 331) geklebt und befestigt ist oder auf der Innenwandfläche des Gehäuses (311, 331) durch Einrasten befestigt ist.

8. Drahtlose Doppelsonden-Ultraschallvorrichtung nach Anspruch 6, wobei die drahtlose Doppelsonden-Ultraschallvorrichtung ferner eine Batterie (90) aufweist, die im Inneren des Sondenkörpers (10) angeordnet ist.

9. Verfahren zur Steuerung einer drahtlosen Doppelsonden-Ultraschallvorrichtung nach Anspruch 1, wobei das Verfahren die folgenden Schritte aufweist:
Überwachen einer aktuell aktivierten Ultraschallsonde der ersten Ultraschallsonde und der zweiten Ultraschallsonde in Echtzeit nach einem Einschalten der drahtlosen Doppelsonden-Ultraschallvorrichtung,
wenn die erste Ultraschallsonde überwacht und aktiviert ist, Einschalten der ersten Antenne und Ausschalten der zweiten Antenne;
wenn die zweite Ultraschallsonde überwacht und aktiviert wird, Einschalten der zweiten Antenne und das Ausschalten der ersten Antenne.

10. System zur Steuerung einer drahtlosen Doppelsonden-Ultraschallvorrichtung nach Anspruch 1, wobei das System Folgendes aufweist:
ein Betriebszustandsüberwachungsmodul (100), das so konfiguriert ist, dass es eine aktuell aktivierte Ultraschallsonde der ersten Ultraschallsonde und der zweiten Ultraschallsonde in Echtzeit überwacht, nachdem die drahtlose Doppelsonden-Ultraschallvorrichtung eingeschaltet wurde;
ein Steuerumwandlungsmodul (200), das so konfiguriert ist, dass es Einschalten einer Antenne der ersten Ultraschallsonde und der zweiten Antenne durch die aktivierte Ultraschallsonde steuert;
wobei, wenn die erste Ultraschallsonde überwacht und aktiviert wird, die erste Antenne eingeschaltet wird und die zweite Antenne ausgeschaltet wird,
wenn die zweite Ultraschallsonde überwacht und aktiviert wird, die zweite Antenne eingeschaltet wird und die erste Antenne ausgeschaltet wird.

## Revendications

1. Dispositif ultrasonore sans fil à double sonde, comprenant : deux ensembles de sondes à ultrasons agencés sur un corps de sonde (10) et présentant des positions d'installation différentes, et une carte de commande (50) connectée en communication avec les deux ensembles de sondes à ultrasons respectivement et agencée à l'intérieur du corps de sonde (10) ;
dans lequel le dispositif ultrasonore sans fil à double sonde comprend en outre : deux ensembles d'antennes pour la transmission de données et connectées en communication avec la carte de commande ;
dans lequel, les deux ensembles de sondes à ultrasons comprennent : une première sonde à ultrasons (31) et une seconde sonde à ultrasons (33) ;
les deux ensembles d'antennes comprenant : une première antenne (71) agencée sur la seconde sonde à ultrasons (33) et connectée en communication avec la première sonde à ultrasons (31), et une seconde antenne (73) agencée sur la première sonde à ultrasons (31) et connectée en communication avec la seconde sonde à ultrasons (33).

2. Dispositif ultrasonore sans fil à double sonde selon la revendication 1, dans lequel un matériau du corps de sonde (10) est un matériau métallique et/ou un matériau plastique.

3. Dispositif ultrasonore sans fil à double sonde selon la revendication 1, dans lequel les deux ensembles de sondes à ultrasons sont respectivement agencés au niveau de deux parties d'extrémité opposées du corps de sonde (10).

4. Dispositif ultrasonore sans fil à double sonde selon la revendication 1, dans lequel au moins l'un des deux ensembles d'antennes est agencé à l'écart du corps de sonde (10).

5. Dispositif ultrasonore sans fil à double sonde selon la revendication 1, dans lequel chaque sonde à ultrasons comprend : un boîtier (311, 331) configuré pour recevoir un composant fonctionnel de la sonde à ultrasons, et au moins l'un des deux ensembles d'antennes est agencé de manière adjacente à une surface de paroi interne du boîtier (311, 331).

6. Dispositif ultrasonore sans fil à double sonde selon la revendication 5, dans lequel au moins l'un des deux ensembles d'antennes est agencé sur la surface de paroi interne du boîtier (311, 331).

7. Dispositif ultrasonore sans fil à double sonde selon la revendication 6, dans lequel au moins l'un des deux ensembles d'antennes est collé et fixé sur la surface de paroi interne du boîtier (311, 331), ou fixé sur la surface de paroi intérieure du boîtier (311, 331) par encliquetage.

8. Dispositif ultrasonore sans fil à double sonde selon la revendication 6, comprenant en outre : une batterie (90) agencée à l'intérieur du corps de sonde (10).

9. Procédé de commande d'un dispositif ultrasonore sans fil à double sonde selon la revendication 1 comprenant :
la surveillance d'une sonde à ultrasons actuellement activée des première et seconde sondes à ultrasons en temps réel après la mise sous tension du dispositif ultrasonore sans fil à double sonde ;
si la première sonde à ultrasons est surveillée et activée, la mise sous tension de la première antenne et la mise hors tension de la seconde antenne ;
si la seconde sonde à ultrasons est surveillée et activée, la mise sous tension de la seconde antenne et la mise hors tension de la première antenne.

10. Système de commande d'un dispositif ultrasonore sans fil à double sonde selon la revendication 1 comprenant :
un module de surveillance d'état de fonctionnement (100), configuré pour surveiller une sonde à ultrasons actuellement activée des première et seconde sondes à ultrasons en temps réel après la mise sous tension du dispositif ultrasonore sans fil à double sonde ;
un module de conversion de commande (200), configuré pour commander la mise sous tension d'une antenne des première et seconde antennes par l'intermédiaire de la sonde à ultrasons activée ;
dans lequel, si la première sonde à ultrasons est surveillée et activée, la première antenne est mise sous tension et la seconde antenne est mise hors tension ;
si la seconde sonde à ultrasons est surveillée et activée, la seconde antenne est mise en sous tension et la première antenne est mise hors tension.
